# EUROPEAN PATENT SPECIFICATION

(11) **EP 2 778 223 B1**
(45) Date of publication and mention of the grant of the patent: **23.11.2016**
(21) Application number: 12847601.7
(22) Date of filing: 07.11.2012
(51) Int. Cl.: C12N 5/071, A61K 35/12, A61L 27/00, A61P 9/00, A61P 9/10, A61P 17/02, C12M 1/00, C12M 3/04, C12N 5/10, A61L 27/52, C12N 5/074, A61L 27/38

(54) **VASCULAR PROGENITOR CELL SHEET DERIVED FROM INDUCED PLURIPOTENT STEM CELLS, AND PRODUCTION METHOD THEREFOR**
VASKULÄRE VORLÄUFERZELLSCHICHT AUS INDUZIERTEN PLURIPOTENTEN STAMMZELLEN UND HERSTELLUNGSVERFAHREN DAFÜR
FEUILLET DE CELLULES PROGÉNITRICES VASCULAIRES ISSU DE CELLULES SOUCHES PLURIPOTENTES INDUITES, ET SON PROCÉDÉ DE PRODUCTION

(30) Priority: 08.11.2011 JP 2011244046
(43) Date of publication of application: 17.09.2014
(73) Proprietor: National University Corporation Nagoya University, Nagoya-shi, Aichi 464-8601 (JP)
(72) Inventor: MUROHARA, Toyoaki, Nagoya-shi Aichi 464-8601 (JP); HONDA, Hiroyuki, Nagoya-shi Aichi 464-8601 (JP); SHIBATA, Rei, Nagoya-shi Aichi 464-8601 (JP); ISHII, Masakazu, Nagoya-shi Aichi 464-8601 (JP); KITO, Tetsutaro, Nagoya-shi Aichi 464-8601 (JP); SUZUKI, Hirohiko, Nagoya-shi Aichi 464-8601 (JP)
(74) Representative: Steinecke, Peter
(86) International application number: PCT/JP2012/078787
(87) International publication number: WO 2013/069661

(56) References cited:
- EP-A1- 1 605 038
- WO-A1-2004/083412
- ITO AKIRA ET AL: "CONSTRUCTION AND HARVEST OF MULTILAYERED KERATINOCYTE SHEETS USING MAGNETITE NANOPARTICLES AND MAGNETIC FORCE", TISSUE ENGINEERING, LARCHMONT, NY, US, vol. 10, no. 5-6, 1 May 2004 (2004-05-01), pages 873-880, XP009081987, ISSN: 1076-3279, DOI: 10.1089/1076327041348446
- SUZUKI HIROHIKO ET AL: "Therapeutic angiogenesis by transplantation of induced pluripotent stem cell-derived Flk-1 positive cells, art 72", BMC CELL BIOLOGY, BIOMED CENTRAL, LONDON, GB, vol. 11, no. 1, 22 September 2010 (2010-09-22), pages 1-10, XP021072067, ISSN: 1471-2121, DOI: 10.1186/1471-2121-11-72
- G. NARAZAKI ET AL: "Directed and Systematic Differentiation of Cardiovascular Cells From Mouse Induced Pluripotent Stem Cells", CIRCULATION, vol. 118, no. 5, 29 July 2008 (2008-07-29) , pages 498-506, XP055067463, ISSN: 0009-7322, DOI: 10.1161/CIRCULATIONAHA.108.769562
- M. ISHII ET AL: "Enhanced Angiogenesis by Transplantation of Mesenchymal Stem Cell Sheet Created by a Novel Magnetic Tissue Engineering Method", ARTERIOSCLEROSIS, THROMBOSIS, AND VASCULAR BIOLOGY, vol. 31, no. 10, 1 October 2011 (2011-10-01), pages 2210-2215, XP055067461, ISSN: 1079-5642, DOI: 10.1161/ATVBAHA.111.231100
- TETSUTARO KITO ET AL: "iPS cell sheets created by a novel magnetite tissue engineering method for reparative angiogenesis", SCIENTIFIC REPORTS, vol. 3, 1 January 2013 (2013-01-01), XP055193301, DOI: 10.1038/srep01418
- SHIMIZU, K. ET AL.: 'Construction of multi-layered cardiomyocyte sheets using magnetite nanoparticles and magnetic force.' BIOTECHNOL. BIOENG. vol. 96, no. 4, 01 March 2007, pages 803 - 809, XP055067453
- ISHII, M. ET AL.: 'Enhanced angiogenesis by transplantation of mesenchymal stem cell sheet created by a novel magnetic tissue engineering method.' ARTERIOSCLER. THROMB. VASC. BIOL. vol. 31, no. 10, October 2011, pages 2210 - 2215, XP055067461
- NARAZAKI, G. ET AL.: 'Directed and systematic differentiation of cardiovascular cells from mouse induced pluripotent stem cells.' CIRCULATION vol. 118, no. 5, 29 July 2008, pages 498 - 506, XP055067463
- SUZUKI, H. ET AL.: 'Therapeutic angiogenesis by transplantation of induced pluripotent stem cell-derived Flk-1 positive cells. art 72' BMC CELL BIOL. vol. 11, 22 September 2010, pages 1 - 10, XP021072067
- HIRASHIMA, M. ET AL.: 'Maturation of embryonic stem cells into endothelial cells in an in vitro model of vasculogenesis.' BLOOD vol. 93, no. 4, 15 February 1999, pages 1253 - 63, XP002963054

## Description

### TECHNICAL FIELD

The present invention relates to a cell sheet, and more specifically to a vascular progenitor cell sheet derived from induced pluripotent stem cells (iPS cells), and a production method therefor. The present application claims the priority based on Japanese Patent Application No. 2011-244046 filed on November 8, 2011, and the entire contents of which are incorporated herein by reference.

### BACKGROUND ART

With the advent of the aging society, the number of patients with ischemic heart disease and obstructive arteriosclerosis is markedly increasing. Usually they are subjected to intravascular treatments such as bypass surgery or catheter, but severe cases who cannot be cured by these treatments are also increasing. For these cases, new treatment "angiogenesis therapy" is used, wherein revascularization and development of collateral circulation are promoted from the tissues around the ischemia part, the blood flow in the ischemia region and surrounding tissues is improved, and thus tissue disorders and necrosis are reduced. The research group including the present inventors started the treatment of critical inferior limb ischemia by marrow monocytes (bone marrow stem cells) (therapeutic angiogenesis by cell transplantation; TACT) on 2000 first in the world, and reported its effectiveness (Non-patent Document 1).

The angiogenesis therapy using bone marrow stem cells showed effectiveness for cardiovascular diseases such as ischaemic heart disease and obstructive arteriosclerosis. However, the therapy has many problems to be solved, such as a heavy burden on the patient due to general anesthesia for collecting bone marrow stem cells, and difficulty in transplantation. In addition, there are many cases suffered from a difficulty in revascularization and blockage of vascular graft. Therefore, finding of a new cell source which replaces bone marrow stem cells, and establishment of highly efficient cell transplantation are required.

In order to fulfill the above requirement, the research group of the present inventors focused on and studied the Flk-1 (fetal liver kinase-1) positive cells induced from iPS cells. The iPS cell-derived Flk-1⁺ cells are also referred to as vascular progenitor cells (VPCs) because they can be differentiated into vascular endothelial cells, vascular smooth muscle cells, and heart muscle cells (Non-patent Document 2). In the results of the previous studies, we report that the Flk-1 (fetal liver kinase-1) positive cells induced from iPS cells promote angiogenesis, and showed usefulness of the cells (Non-patent Document 3). On the other hand, from the viewpoint of allowing efficient and effective cell transplantation, we focused on a cell sheet, and attempted to construct a sheet of iPS cell-derived Flk-1⁺ cells. Specifically, we tested a method for allowing Flk-1⁺ cells to take in magnetic particles, and culturing the cells under a magnetic force, and a method for isolating the Flk-1⁺ cells using MACS (magnetic cell separation) or FCM (flowcytometry), and then culturing the cells. However, no practicable cell sheet was obtained. On the other hand, as a result of the combination with the adipose tissue-derived stem cells (ADRCs), which are receiving attention as a cell source, a two-layer sheet (a sheet of iPS cell-derived Flk-1⁺ cells is overlaid on an ADRC sheet) was successfully constructed, but only a very weak sheet was obtained when the ADRC and iPS cell-derived Flk-1⁺ cells were mixed in a mosaic pattern.

### PRIOR ART DOCUMENT

### NON-PATENT DOCUMENT

Non-patent Document 1: Tateishi-Yuyama E. et al., Lancet. 2002 Aug 10; 360 (9331): 427-35.
Non-patent Document 2: Narazaki G. et al., Circulation. 2008 Jul 29; 118 (5): 498-506.
Non-patent Document 3: Suzuki et al., BMC Cell Biology 2010, 11: 72

### SUMMARY OF THE INVENTION

### PROBLEM TO BE SOLVED BY THE INVENTION

In order to allow transplantation, and to achieve high therapeutic effect, the cell sheet must have a sufficient strength. As described above, the iPS cell-derived Flk-1⁺ cells promote angiogenesis (see, for example, Non-patent Document 3), and is highly expected to find application in the treatment of ischemic diseases and wounds. However, the application to a cell sheet is very difficult, so that the construction of a cell sheet having a sufficient strength has not been achieved. Accordingly, the present invention is intended to provide a sheet of iPS cell-derived Flk-1⁺ cells (vascular progenitor cells) which has a practically sufficient strength, and achieves high therapeutic effect.

### MEANS FOR THE SOLVING THE PROBLEM

During the study, the inventors focused on the magnetic engineering technology and collagen embedding method, and combined them to develop an original method for constructing a cell sheet. More specifically, they developed a method for forming a cell layer by mixing a gel including collagen and basement membrane components with the magnetically labeled iPS cell-derived Flk-1⁺ cells, and then moving the cells using magnetic force. The effectiveness of the method was studied, and the construction of a sheet with a sufficient strength composed solely of the iPS cell-derived Flk-1⁺ cells was achieved. The sheet has a structure composed of multilayers (about 10 to 15 layers) of Flk-1⁺ cells, and showed a sufficient strength for transplantation. In addition, the therapeutic effect was validated by transplanting the sheet into a model with limb ischemia; good adhesion and integration were exhibited, and marked improvement in ischemia was achieved. More specifically, the achievement of high therapeutic effect was confirmed. In addition, appropriate gaps are formed in the cell sheet between the cells (a gel intervenes between the cells), which allows angiogenesis in the sheet after transplantation. This characteristic is considered to contribute to the improvement of transplantation efficiency and integration ratio.

As described above, the inventors studied based on their unique viewpoint, and have succeeded in the development of an epoch-making method for constructing a "cell sheet" which is important for clinical application of the iPS cell-derived Flk-1⁺ cells. The following aspects of the present invention are based mainly on the result of the study.
[1] A method for producing an iPS cell-derived vascular progenitor cell sheet, including the following steps (1) to (4):
   (1) a step for preparing magnetically labeled iPS cell-derived Flk-1⁺ cells;
   (2) a step for plating a mixture of a gel material comprising type I collagen, laminin, type IV collagen, and entactin as active ingredients, and the Flk-1⁺ cells in a culture vessel;
   (3) a step for drawing the Flk-1⁺ cells in the mixture to the culture surface in the culture vessel by application of a magnetic force to form multiple cell layers; and
   (4) a step for gelling the gel material.
[2] The production method of [1], wherein the step (1) includes the following steps (1-1) to (1-4):
   (1-1) a step for preparing iPS cells;
   (1-2) a step for inducing differentiation of the iPS cells into Flk-1⁺ cells;
   (1-3) a step for collecting Flk-1⁺ cells; and
   (1-4) a step for magnetically labeling the collected Flk-1⁺ cells.
[3] The production method of [2], wherein in the step (1-3), Nanog⁺ cells and Nanog⁻ cells are separated, and the Nanog⁻ Flk-1⁺ cells are collected.
[4] The production method of any one of [1] to [3], wherein the mixture in the step (2) is obtained by mixing a first gel element composed of type I collagen as an active ingredient, a second gel element composed of laminin, type IV collagen, and entactin as active ingredients, and the Flk-1⁺ cells.
[5] The production method of any one of [1] to [4], wherein an upwardly open section made by removable partitions is formed on the culture surface in the culture vessel in the step (2), and the mixture is plated in the section.
[6] The production method of any one of [1] to [5], wherein the culture surface is low-adhesive.
[7] The production method of any one of [1] to [6], wherein the step (3') is carried out between the steps (3) and (4):
   (3') a step for removing the redundant part of the gel material from the upper part of the cell layer.
[8] The production method of any one of [1] to [7], wherein the following step (5) is carried out after the step (4):
   (5) a step for adding a medium to the culture vessel, and maintaining the sheet-like structure formed by the step in the medium.
[9] The production method of [8], wherein the following step (6) is carried out after the step (5):
   (6) a step for culturing the Flk-1⁺ cells under temperature conditions which allow their growth.
[10] A cell sheet obtained by the production method of any one of [1] to [9].
[11] A cell sheet composed of multiple layers of iPS cell-derived Flk-1⁺ cells embedded in a gel containing type I collagen, laminin, type IV collagen, and entactin.
[12] The cell sheet of [11], wherein the gel is present between the cells forming the multiple layers.
[13] The cell sheet of [11] or [12], wherein the multiple layers include at least 10 layers.
[14] The cell sheet of [11] or [12], wherein the multiple layers include 10 to 20 layers.
[15] The cell sheet of any one of [11] to [14], wherein the cell component contained in the multiple layers is composed solely of iPS cell-derived Flk-1⁺ cells.
[16] The cell sheet of any one of [11] to [14], wherein the cell component contained in the multiple layers is composed solely of the iPS cell-derived Flk-1⁺ cells and the cells derived from the cells.
[17] The cell sheet of any one of [11] to [16], wherein the iPS cell-derived Flk-1⁺ cells forming the multiple layers are magnetically labeled.
[18] The cell sheet of any one of [11] to [17], wherein the iPS cell-derived Flk-1⁺ cells are Nanog⁻ cells.
[19] An angiogenesis therapy including a step for transplanting the cell sheet of any one of [10] to [18] to the affected or injury part.
[20] The angiogenesis therapy of [19], which is used for healing of ischemic heart disease, cerebrovascular disorder, obstructive arteriosclerosis, critical inferior limb ischemia, or wound, or postoperative healing of wound.

### BRIEF DESCRIPTION OF DRAWING

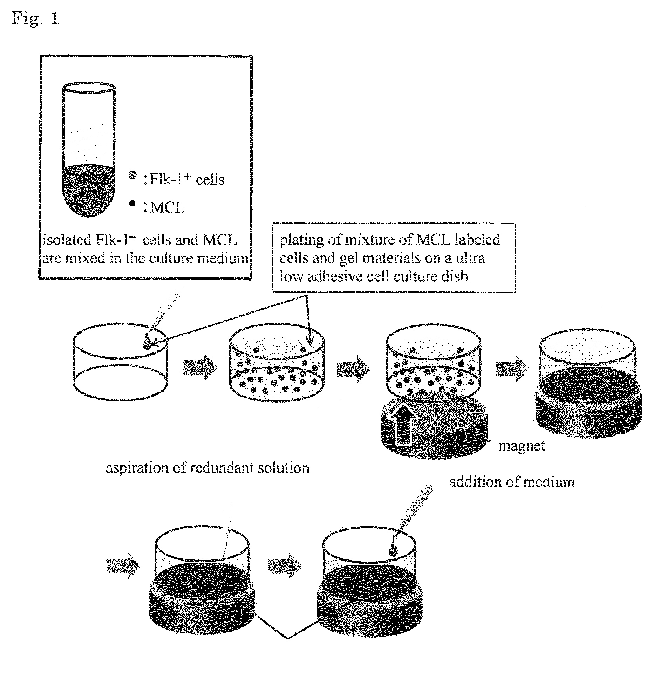

Fig. 1 is an example of the method for producing an iPS cell-derived FLk-1⁺ cell sheet.

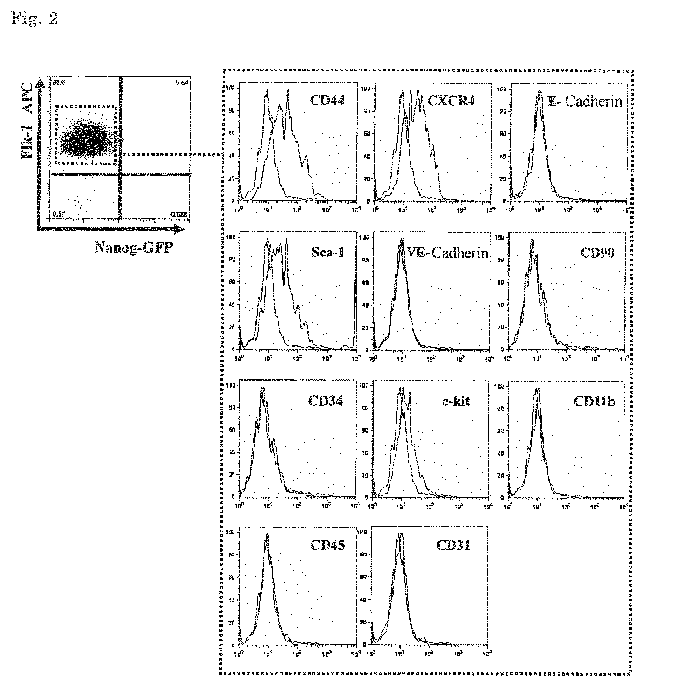

Fig. 2 is the result of FCM (flowcytometry) analysis of the Flk-1⁺ Nanog⁻ cells differentiated from iPS cells. The Flk-1⁺ Nanog⁻ cells were collected, and the expression of various cell surface markers was detected.

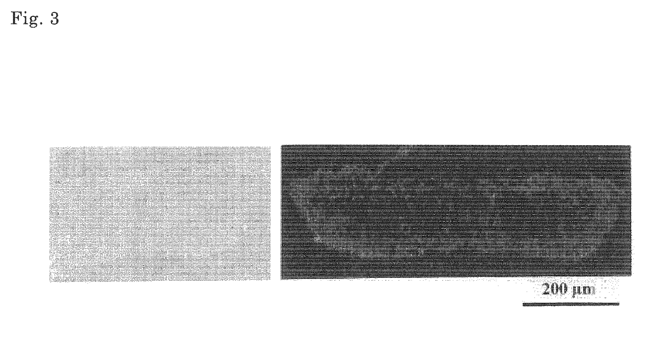

Fig. 3 shows the iPS cell-derived FLk-1⁺ cell sheet successfully produced. The cross section was observed using an optical microscope and a fluorescence microscope. The left is a bright field image, and the right is a fluorescence microscope image (Flk-1/DAPI). It is indicated that Flk-1⁺ cells forms 10 to 15 cell layers. The expression of CD31 or αSMA was fond in some cells (data not shown).

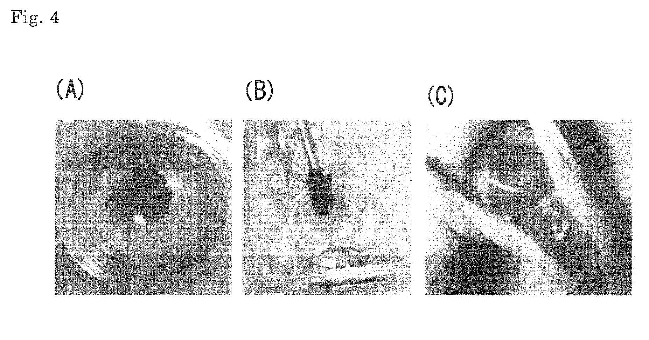

Fig. 4 shows the iPS cell-derived Flk-1⁺ cell sheets (A and B) produced and the iPS cell-derived FLk-1⁺ cell sheet after transplantation. The cell sheet had flexibility and a sufficient strength (B), and exhibited good adhesiveness (C).

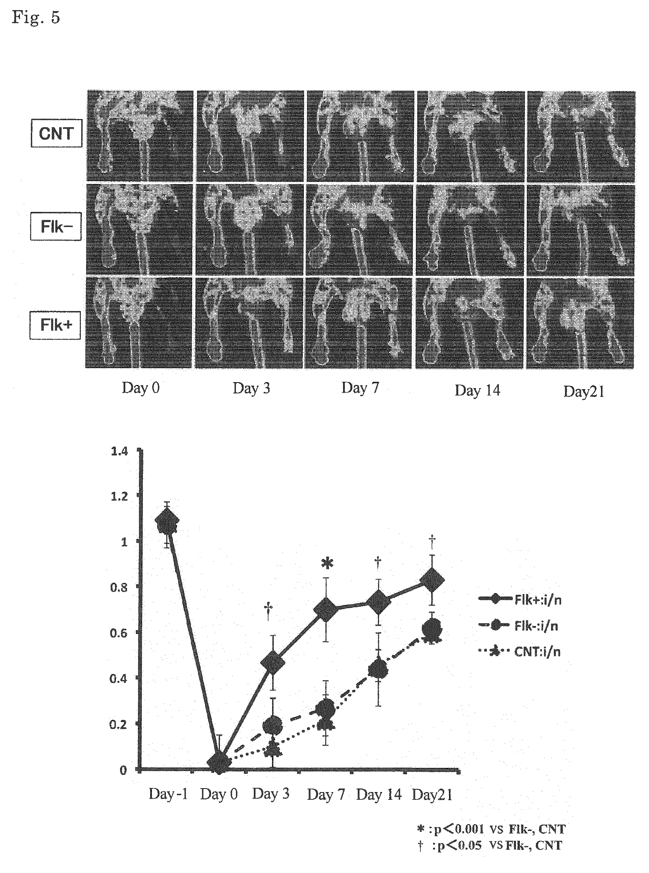

Fig. 5 shows the evaluation of the angiogenesis capability of the iPS cell-derived FLk-1⁺ cell sheet. The cell sheet was transplanted to a model with limb ischemia, and the blood flow was detected over time by the laser Doppler method. The iPS cell-derived FLk-1⁺ cell sheet (Flk⁺) transplant group showed a significant improvement in the blood flow on the limb ischemia side in comparison with the iPS cell-derived Flk-1⁻ cell sheet transplant group (Flk⁻) and the control group (CNT). The lower graph shows the comparison of the blood flow ratio (ordinate) between the ischemic and healthy sides.

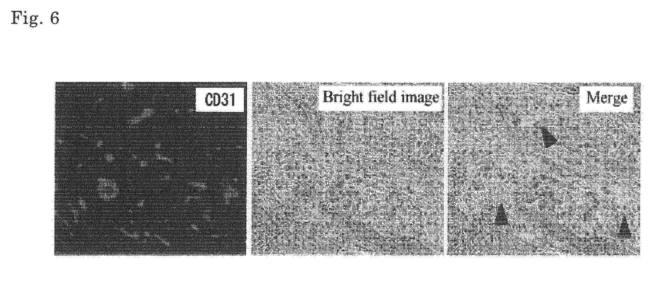

Fig. 6 shows the characteristic of the iPS cell-derived FLk-1⁺ cell sheet 21 days after transplantation. A fluorescence microscopic image (left), a bright field image (center), and the synthesis of them (right) are shown. Many new blood vessels (arrow) are found.

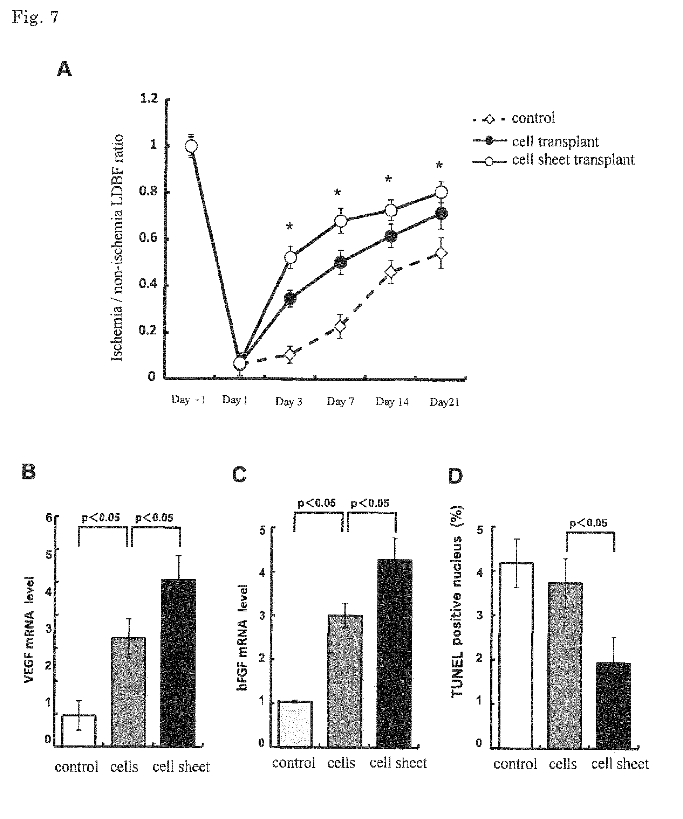

Fig. 7 shows the therapeutic effect of the iPS cell-derived FLk-1⁺ cell sheet. After transplantation of the cell sheet to a model with limb ischemia, the blood flow was detected over time by the laser Doppler method. The iPS cell-derived FLk-1⁺ cell sheet transplant group (Flk⁺) showed a significant improvement in the blood flow on the limb ischemia side in comparison with the cell transplant group (A). The symbol * indicates the presence of a significant difference. The VEGF mRNA level (b), bFGF mRNA level (c), and TUNEL positive ratio (d) were also compared.

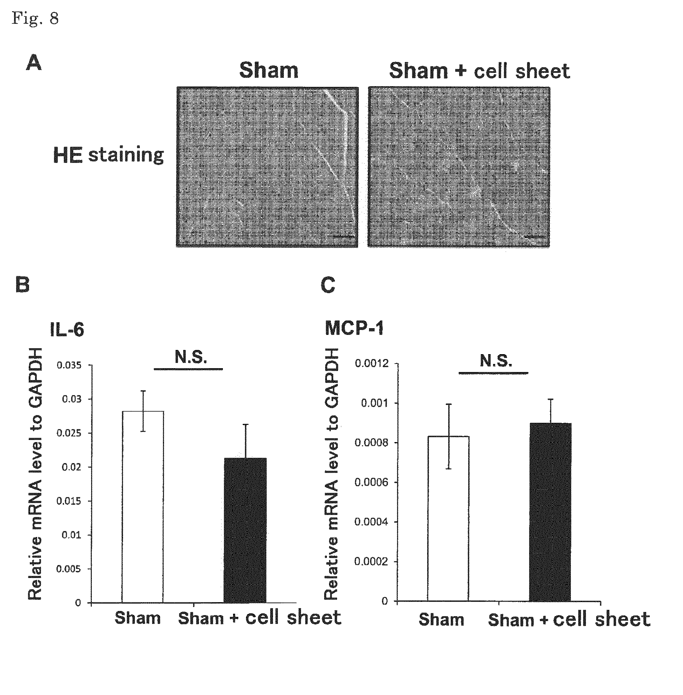

Fig. 8 shows the study of rejection of the iPS cell-derived FLk-1⁺ cell sheet. The iPS cell-derived FLk-1⁺ cell sheet was transplanted to the adducent muscles of lower limbs of a wild type mouse of C57/BL6 strain, and the presence or absence of rejection was studied. The symbol A indicates the result of staining with hematoxylin eosin (HE). The left shows the SHAM group, and the right shows the iPS cell-derived FLk-1⁺ cell sheet transplant group. The scale bar is 50.0 µm. The expression level of inflammatory cytokine (B: IL-6, C: MCP-1) was compared by real time RT-PCR method. The mRNA level of each cytokine was expressed by the relative value (vs. GAPDH mRNA level). N.S. means no significant difference.

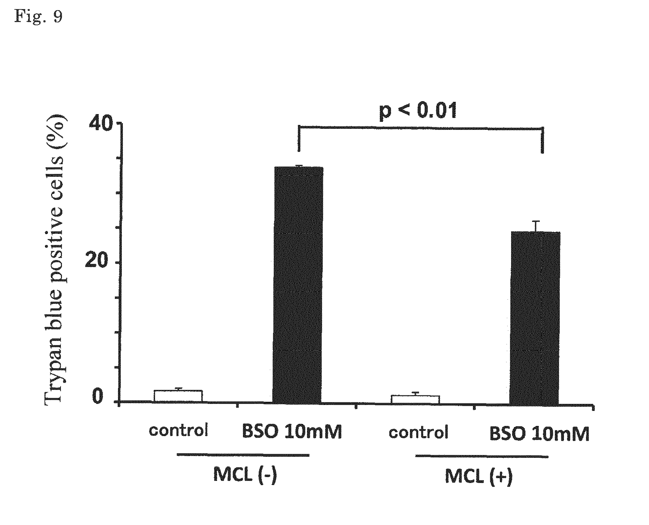

Fig. 9 shows the comparison of the number of dead cells between the magnetically labeled iPS cell-derived Flk-1⁺ cells (MCL(+)) and the iPS cell-derived Flk-1⁺ cells (MCL(-)) before magnetic labeling using trypan blue staining. After treating these cells with BSO, and subjected to trypan blue staining.

### DESCRIPTION OF EMBODIMENT

### 1. Method for producing an iPS cell-derived vascular progenitor cell sheet

A first aspect of the present invention relates to a method for producing an iPS cell-derived vascular progenitor cell sheet. The "iPS cells" are the cells having pluripotency and proliferation potency which are prepared by reprogramming the somatic cells by, for example, the introduction of an initialization factor. The properties of the iPS cells are close to those of embryonic stem cells (ES cells).

The "iPS cell-derived vascular progenitor cells" are the Flk-1⁺ cells obtained by inducing differentiation of iPS cells. According to the production method of the present invention, a cell sheet composed of multiple layers of Flk-1⁺ cells is obtained.

The production method of the present invention includes the following steps (1) to (4):
(1) a step for preparing magnetically labeled iPS cell-derived Flk-1⁺ cells;
(2) a step for plating a mixture of a gel material comprising type I collagen, laminin, type IV collagen, and entactin as active ingredients, and the Flk-1⁺ cells in a culture vessel;
(3) a step for drawing the Flk-1⁺ cells in the mixture to the culture surface in the culture vessel by application of a magnetic force to form multiple cell layers; and
(4) a step for gelling the gel material.

### <Step (1): preparation of magnetically labeled cells>

In the step (1), magnetically labeled iPS cell-derived Flk-1⁺ cells are provided. The term "magnetic labeling" has the same meaning as "magnetization", and refer to the introduction or adhesion of magnetic particles to cells, thereby allowing the operation of the cell by a magnetic force. Magnetic labeling of cells is achieved preferably by the introduction or adhesion of magnetic particles. Magnetic particles are any particles as long as they can be held by cells, and impart and can magnetize the cells holding them. For example, the magnetic particles may be the particles of a magnetic material such as iron oxide including ferrite and magnetite, chromic oxide, and cobalt. Two or more magnetic particles may be combined. The particle size of the magnetic particles is not particularly limited, and may be, for example, from 5 nm to 100 µm. For the below-described magnetic particles encapsulated in liposome, the particle size of the magnetic particles is preferably from 5 nm to 25 nm. When the particle size of the magnetic particles is within this range, dispersion stability of liposome is improved.

When the cells are magnetically labeled by the introduction of magnetic particles, the magnetic particles which have been prepared to have a form suitable for the introduction into the cells is used. A specific example of the magnetic particles having this form is the magnetic particles encapsulated in lipid membrane such as liposome. For example, magnetoliposome or magnetite liposome (ML) prepared by encapsulating magnetic particles in liposome, or magnetite cationic liposome (MCL) prepared by encapsulating magnetic particles in cationic liposome may be used. These magnetic particles in encapsulated in liposome are adhered to and taken into the cells by the affinity of liposome for the cells. In particular, the MCL is efficiently taken into the cells by hydrophobic interaction or electrical interaction with the cell surface. The intake of magnetic particles into the cells allows more reliable magnetic labeling of the cells, and many magnetic particles are held by the cells, so that the cells can be easily controlled by the action of magnetic force.

Specific example of MCL include the magnetic particles such as magnetite encapsulated in liposome containing cationic lipid. This MCL has a cationic surface, and thus has good adhesion to the cells, and is readily taken into the cells because it is composed of liposome. The MCL having these properties is suitable for magnetic labeling of various cells. MCL can be prepared by, for example, with reference to the method for producing MCL described in Jpn. J. Cancer Res. Vol. 87, pages 1179 to 1183 (1996).

On the other hand, when the cells are magnetically labeled by adhering magnetic particles thereto, the magnetic particles are preferably a complex with a cell adhesion substance. For example, magnetic particles can be adhered to the cells by using a complex composed of a cell adhesion substance directly or indirectly bonded to magnetic particles, or a complex composed of magnetic particles coated with or encapsulated in a material containing a cell adhesion substance (for example, polysaccharide or lipid). The above-described magnetic particles encapsulated in liposome also adhere to cells, and may be used for magnetic labeling by the adhesion of magnetic particles.

The cell adhesion substances can be classified into the substances having adhesion to a wide range of cells, and those having selective adhesion to specific cells. Examples of the former one include the compound which bonds or adheres to the components of a cell membrane. Examples of the compound include fibronectin, peptide which is a part of fibronectin and containing an amino acid sequence such as RGD (Arg-Gly-Asp, arginine-glycine-asparatic acid), KQAGDV (Lys-Gln-Ala-Gly-Asp-Val, ricin-glutamine-alanine-glycine-asparatic acid-valine) (SEQ ID NO.1) or REDV (Arg-Glu-Asp-Val, arginine-glutamic acid-asparatic acid-valine) (SEQ ID NO.2), laminin which is also a cell adhesion protein, and a peptide which is a part of laminin and containing an amino acid sequence such as YIGSR (Tyr-Ile-Gly-Ser-Arg, tyrosine-isoleucine-glycine-serine-arginine) (SEQ ID No. 3), or iKVAV (Ile-Lys-Val-Ala-Val, isoleucine-ricin-valine-alanine-valine) (SEQ ID No. 4). The length of the cell adhesion peptide is not particularly limited, and is preferably several to ten several amino acids, and even more preferably about 10 or less amino acids. For example, the peptide is preferably a peptide having an amino acid sequence RGD or a peptide having an amino acid sequence yigsr (SEQ ID No. 3) and an amino acid residue number of 10 or less. The cell adhesion peptide preferably has any of these specific amino acid sequences on the terminal side of the peptide, and is more preferably bonded to the surface of, for example, magnetic particles at the C terminal side with the amino acid sequence located at the N terminal side. Even more preferably, the N terminal residue of the amino acid sequence is located at the N terminal.

On the other hand, examples of the substance which has selective adhesion to specific cells include the antibody against the molecule (marker molecule) on whose surface specific cells are expressed. The antibody may be an antibody fragment such as Fab, Fab', F(ab')₂, scFv, and dsFv. A fusion antibody or labeled antibody composed of a low molecular weight compound, a protein, or a labeling agent fused or bonded together. Examples of the labeling agent include a radioactive material such as ¹²⁵I, peroxidase, β-D-galactosidase, microperoxidase, horseradish peroxidase (HRP), fluorescein isothiocyanate (FITC), rhodamine isothiocyanate (RITC), alkaline phosphatase, and biotin.

Cell adhesion magnetic particles are constructed by bonding a cell adhesion substance directly or indirectly to magnetic particles. For example, cell adhesion magnetic particles can be obtained by bonding an antibody to commercially available magnetic particles dynabeads (registered trademark) using the binding reaction between biotin and streptavidin. Alternatively, cell adhesion magnetic particles bonded to a cell adhesion substance can be constructed by amino silane coupling of commercially available magnetic particles RESOVIST (registered trademark), or FERIDEX. Alternatively, cell adhesion magnetic particles can be constructed by encapsulating magnetic particles in liposome having a cell adhesion substance on its surface (more specifically, liposome containing a cell adhesion substance or liposome whose surface has a cell adhesion substance adhered or bonded thereto). The magnetite liposome can be made by using various kinds of bonding reaction according to the type of the cell adhesion substance. As necessary, an appropriate linker may be used. For example, a method using the formation of a disulfide bond is preferred for bonding an RGD peptide to liposome. This method preferably uses a peptide having an RGDC sequence (SEQ ID No. 5) composed of an RGD sequence whose C terminal has cysteine. The use of this peptide allows easy formation of a disulfide bond with the liposome side having SH groups. The linker for bonding a cell adhesion peptide to liposome is not limited to cysteine, but may be other amino acid or peptide.

Specific examples of the magnetic particles forming a composite with a cell adhesion substance (cell adhesion magnetic particles) include magnetite liposome composed of MCL whose liposome surface is bonded to a peptide having an amino acid sequence RGDC (SEQ ID No. 5). Other specific examples of the cell adhesion magnetic particles include antibody-immobilized magnetite liposome (AML) obtained by bonding an antibody to the liposome surface of MCL. AML is composed of magnetic particles such as magnetite encapsulated in liposome, and an antibody immobilized on the liposome. The antibody is chosen from those specifically bond to the cells to be magnetically labeled. As a result of this, specific cells are magnetically labeled. AML can be prepared with reference to, for example, the method described in J. Chem. Eng. Jpn. Vol. 34, pages 66 to 72 (2001).

The magnetically labeled iPS cell-derived Flk-1⁺ cells can be prepared by, for example, a method for inducing iPS cells to differentiate into Flk-1⁺ cells, and then collecting the Flk-1⁺ cells and subjecting to magnetic labeling, a method for inducing iPS cells to differentiate into Flk-1⁺ cells, magnetically labeling them, and then collecting the Flk-1⁺ cells, or a method for magnetically labeling the iPS cells, inducing them to differentiate into Flk-1⁺ cells, and then collecting Flk-1⁺ cells. A specific example of the method for preparing the magnetically labeled iPS cell-derived Flk-1⁺ cells is described below. This example includes the following steps, more specifically, (1-1) a step of providing iPS cells; (1-2) a step of inducing the iPS cells to differentiate into Flk-1⁺ cells; (1-3) a step of collecting Flk-1⁺ cells; and (1-4) a step of magnetically labeling the collected Flk-1⁺ cells.

Firstly, iPS cells are provided (step (1-1)). The iPS cells can be prepared by any of the various reported methods for preparing iPS cells. In addition, it is aslo contemplate to use the method for preparing iPS cells to be developed in future. A basic method for preparing iPS cells is the method for introducing the four factors, or Oct3/4, Sox2, Klf4, and C-MYC, which are transcription factors, into the cells using a virus (Takahashi K, Yamanaka S: Cell 126 (4), 663-676, 2006; Takahashi, K, et al: Cell 131 (5), 861-72, 2007). For human iPS cells, establishment by the introduction of four factors, or Oct4, Sox2, Lin28, and Nonog is reported (Yu J, et al: Science 318 (5858), 1917-1920, 2007). The establishment of iPS cells by the introduction of three factors excluding C-Myc (Nakagawa M, et al: Nat. Biotechnol. 26 (1), 101-106, 2008), two factors, or Oct3/4 and Klf4 (Kim J B, et al: Nature 454 (7204), 646-650, 2008), or Oct3/4 alone (Kim J B, et al: Cell 136 (3), 411-419, 2009) is also reported. In addition, a method for introducing protein, which is an expression product of gene, into cells is also reported (Zhou H, Wu S, Joo JY, et al: Cell Stem Cell 4, 381-384, 2009; Kim D, Kim CH, Moon Ji, et al: Cell Stem Cell 4, 472-476, 2009). On the other hand, there is a report that the improvement of the preparation efficiency and reduction of the number of the factors to be introduced can be achieved by the use of, for example, an inhibitor BIX-01294 for the histonemethyltransferase G9A, histone deacetylase inhibitor valproic acid (VPA), or BayK8644 (Huangfu D, et al: Nat. Biotechnol. 26 (7), 795-797, 2008; Huangfu D, et al: Nat. Biotechnol. 26 (11), 1269-1275, 2008; Silva J, et al: Plos. Biol. 6 (10), E 253, 2008). Gene introduction methods are also studied, and gene introduction techniques using a retrovirus lentivirus (Yu J, et al: Science 318(5858), 1917-1920, 2007), an adenovirus (Stadtfeld M, et al: Science 322 (5903), 945-949, 2008), plasmid (Okita K, et al: Science 322 (5903), 949-953, 2008), a transposon vector (Woltjen K, Michael IP, Mohseni P, et al: Nature 458, 766-770, 2009; Kaji K, Norrby K, Pac A A, et al: Nature 458, 771-775, 2009; Yusa K, Rad R, Takeda J, et al: Nat Methods 6, 363-369, 2009), or an eposomal vector (Yu J, Hu K, Smuga-Otto K, Tian S, et al: Science 324, 797-801, 2009) are developed.

The transformation into iPS cells, or the initialized (reprogrammed) cells can be selected using, for example, the expression of a pluripotent stem cell marker (undifferentiated marker) such as Fbxo15, Nanog , Oct/4, FGF-4, ESG-1, and CRIPT as the indicator. The selected cells are collected as iPS cells.

In the step (1-2) following the step (1-1), the prepared iPS cells are induced to differentiate into Flk-1⁺ cells. The inductive differentiation of the iPS cells into Flk-1⁺ cell can be carried out in accordance with the method described in a previous report (Narazaki G, Uosaki H, Teranishi M, Okita K, Kim B, Matsuoka S, Yamanaka S, Yamashita J: Directed and Systematic Differentiation of Cardiovascular Cells from Mouse iPS cells. Circulation 2008, 118: 498-506.). In brief, using a differentiation-inducing medium (for example, α-MEM mixed with 10% FBS and 5 x 10⁻⁵ mol/l 2-mercaptoethanol (minimum essential medium)), iPS cells are cultured on a culture dish coated with type IV collagen for a predetermined time (for example, 96 to 108 hours). The inductive differentiation conditions are modified or changed as necessary according to the origin and condition of the iPS cells used. The adequate inductive differentiation conditions can be established based on, for example, preliminary experiments, with reference to the contents of the present description and references.

Subsequently, the Flk-1⁺ cells formed by the inductive differentiation are collected (step (1-3)). The Flk-1⁺ cells are preferably collected by, but not limited to, flow cytometry (FCM). The apparatus for FCM (cell sorter) can be purchase from, for example, Beckman Coulter Inc. and Japan Becton, Dickinson and Company, and the present invention may use these apparatus. The basic operation method and analysis conditions may follow the instruction manual attached to the apparatus. In addition, there are many literatures and publications regarding FCM, and examples of references include Darzynkiewicz Z, Crissman HA, Robinson Jp (eds.): Flow Cytometry. 3rd Edition. Methods in Cell Biology, Volumes 63 (Part A) and 64 (Part B). San Diego, Academic Press, 2000.; Givan AL: Flow Cytometry: First Principles. 2nd Edition. New York, Wiley-Liss, 2001.; Ormerod MG (ed.): Flow Cytometry - A Practical ApproacH. 3rd Edition. Oxford, Oxford University Press, 2000.; Robinson JP, Darzynkiewicz Z, Dean P, Dressler L, Rabinovitch P, Stewart C, Tanke H, Wheeless L, (eds.): and Current Protocols in Cytometry, New York, John Wiley & Sons (continuing updates).

When Flk-1⁺ cells are collected, according to a preferred manner, Nanog⁺ cells and Nanog⁻ cells are separated, and then only the Nanog⁻ Flk-1⁺ cells are collected. The selection of the cells without the expression of a undifferentiated marker Nanog is preferred from the viewpoint of improvement of safety of the cell sheet obtained by the production method of the present invention. More specifically, the use of the Nanog⁻ Flk-1⁺ cell alone is effective for the prevention of tumorigenesis associated with the transplantation of the cell sheet. The selection of the Nanog⁺ cells and Nanog⁻ cells, and collection of the Nanog⁻ Flk-1⁺ cells may use, for example, a cell sorter.

The collected Flk-1⁺ cells are magnetically labeled (step (1-4)). The method for magnetic labeling is as described above. For example, the collected Flk-1⁺ cells are suspended and floated, MCL is added to the culture solution, and the culture solution is incubated for a predetermined time (for example, 2 to 4 hours). As a result of this, Flk-1⁺ cell encapsulating MCL, more specifically magnetically labeled Flk-1⁺ cells are obtained.

### <Step (2): mixing of cells and gel material>

In the step (2) following the step (1), a mixture of a gel material and the Flk-1⁺ cells is plated in a culture vessel. In the present invention, the gel material is composed of type I collagen which is a main component of stroma, laminin composing the basement membrane, type IV collagen, and entactin are used. The active ingredients composing the gel material (type I collagen, laminin, type IV collagen, and entactin) may be derived from horse, bovine, swine, sheep, monkey, chimpanzee, and human. Alternatively, recombinant products prepared by gene recombination technology may be used.

The proportions the active ingredients composing the gel material are not particularly limited. The weight ratio between the active ingredients is, for example, collagen I : laminin : collagen IV : entactin = 1 : 10 to 200 : 5 to 100 : 1 to 50. Preferably, collagen I : laminin : collagen IV : entactin = 1 : 20 to 100 : 10 to 50 : 2 to 25.

The gel material contains the medium components necessary for the living and maintenance of cells. Examples of the medium include Dulbecco's modified Eagle's medium (DMEM) (for example, Nacalai Tesque, Inc., Sigma Corporation, and Gibco), RPMI 1640 medium (for example, Nacalai Tesque, Inc., Sigma Corporation, and Gibco), and SMGM medium (Cambrex Corporation). The gel material may contain, in addition to the medium component, other gelation components (for example, type III collagen and type VIII collagen), cell adhesion factors (for example, fibronectin), blood serum (for example, FBS and human blood serum), and cell growth factors (for example, EGF, PDGF, IGF-1, and TGF-β), differentiation-inducing factors, inorganic salts, vitamins, preservatives, and antiseptics.

According to a preferred embodiment, a first gel element including type I collagen as an active ingredient, and a second gel element including laminin, type IV collagen, and entactin as active ingredients are prepared in advance, and these gel elements and Flk-1⁺ cells are mixed to obtain a mixture of the gel material and Flk-1⁺ cells. For example, the first gel element may be prepared by dissolving type I collagen in a medium, a buffer solution (for example, phosphate buffer solution), or a normal saline solution, and diluting the solution. The second gel element may be prepared by the same method. Alternatively, a commercially available reagent containing the above-described active ingredients (laminin, type IV collagen, and entactin) (for example, a basement membrane matrix such as BD Matrigel™ sold by Japan Becton, Dickinson and Company) may be used. The proportions of the active ingredients in the second gel is not particularly limited, and preferably the proportion (weight ratio) is laminin : type IV collagen : entactin = 3 to 15 : 2 to 8 : 1.

A cell sheet achieving a high transplantation efficiency and an integration rate can be obtained by adjusting the density of the Flk-1⁺ cells such that appropriate gaps are formed by the gel components between the cells. Therefore, the number of the Flk-1⁺ cells to be used is preferably adjusted such that the cell density in the mixture is, for example, from 1.0 x 10⁵ cells/cm³ to 1.0 x 10⁷ cells/cm³, preferably from 1.0 x 10⁶ cells/cm³ to 5.0 x 10⁶ cells/cm³.

The culture vessel to which the mixture of the gel material and Flk-1⁺ cells is plated is not particularly limited. More specifically, various culture vessels may be used. Preferably, a culture vessel opened upward, such as a culture dish (for example, culture dish, multi-well plate). On the other hand, in order to facilitate the recovery of the cell sheet to be formed, the use of a culture vessel having a low adhesive culture surface is preferred. The term "low adhesive culture surface" means a culture surface to which cells are hardly adhered, the culture surface being uncoated or treated with a non-adhesive or low-adhesive material, in contrast to a culture surface coated with polylysine or the like to improve adhesion to cells. Various culture vessels having a low adhesive culture surface are commercially available. For example, Ultra Low Attachment Culture Dish (Corning Incorporated) which is an ultra low adhesive cell culture dish, a culture dish coated with an agarose gel or alginic acid gel, or a culture dish for culturing floating cells may be used.

According to one embodiment of the present invention, an upwardly open section is formed by removable partitions on the culture surface, and a mixture of the gel materials and Flk-1⁺ cells are plated in the section. In this embodiment, the cells are enclosed in the limited region, so that the size of the cell sheet to be obtained finally will not depend on the size of the culture surface. Accordingly, the size of the cell sheet can be freely designed irrespective of the size of the culture surface. In addition, the shape of the cell sheet depends on the shape of the section (for example, a ring shape), and the cell sheet can be provided in various shapes. More specifically, the flexibility of design of the shape of the cell sheet is markedly increased. Furthermore, the use of the section allows the adjustment of the cell density of the cell layers composing the cell sheet.

### <Step (3): Formation of cell layer by magnetic force>

After plating the mixture of the gel material and Flk-1⁺ cells to a culture vessel, for example, magnetic force is applied from the back of the culture surface (more specifically, the backside of the culture surface), thereby drawing the Flk-1⁺ cells in the mixture toward the culture surface. Specifically, for example, a magnet is placed at the back of the culture surface, and this operation is carried out. When a culture dish is used as the culture vessel, typically, the culture dish is placed on the magnet. When a culture dish is used, usually, the inner bottom surface is the culture surface, but the inner wall surface other than the inner bottom surface may be used as the culture surface according to the type and form of the vessel.

The type of the magnet is not particularly limited. For example, a permanent magnet or electromagnet may be used. When an electromagnet is used, the magnetic force can be controlled by the manipulation of the energization condition. Examples of the permanent magnet include casting magnets (including alnico magnet and iron-chromium-cobalt magnet), plasticized magnets (including Fe-Mn magnet and Fe-Cr-Co magnet), ferrite magnets (including Ba magnet and Sr magnet), rare earth magnets (including Sm-Co magnet and Nd-Fe-B magnet), and bond magnets (including Sm-Co magnet, Nd-Fe-B magnet, and Sm-Fe-N magnet).

The time of the application of the magnetic force is not particularly limited as long as multiple cell layers are formed. The application time may be established in consideration of the type of the magnet to be used, the type of the magnetic particles used for magnetic labeling, and the amount and density of the magnetically labeled cells. For example, the magnetic force is applied for 30 minutes to 2 hours. The optimum application time may be established based on a preliminary experiment. A cell layer having a desired thickness and/or a desired cell density can be formed by adjusting the intensity of the magnetic force and application time.

In place of directly using the magnetic force release from the magnet, the magnetic force released from the magnet may be used after transmitted to other member. For example, by bringing a magnet into contact with or close to a member which transmits magnetic force, such as Fe, Co, Ni, Fe-C, Fe-Ni, Fe-Co, Fe-Ni-Co-Al, Fe-Ni-Cr, SmCo₅, Nd₂Fe₁₄B, Fe₃O₄, γ-Fe₂O₃, or BaFe₁₂O₁₉, magnetic force is released from the surface (for example, end surface) of the member.

According to one embodiment of the present invention, the redundant part of the gel material (more specifically, the supernatant liquid) is removed from the upper part of the cell layer formed (step (3')). When this operation is carried out, a cell sheet having no redundant gel layer on the cell layers will be obtained. This cell sheet is advantageous from the viewpoints of handling and therapeutic effect. The removal of the gel material can be carried out by, for example, using an aspirator such as a dropper.

### <Step (4): gelation>

Subsequently, the gel material is gelated. In a typical manner, the gel material is incubated together with the culture vessel at a temperature necessary for gelation (for example, 37°C). The time necessary for gelation depends on the constitution of the gel material and the scale of operation, and is, for example, from 30 minutes to 1 hour.

The sheet-like structure formed by gelation may be collected immediately, but in a preferred manner, the medium is added to the culture vessel, and the sheet-like structure is kept in the medium. The addition of this operation (step (5)) prevents the quality deterioration of the sheet-like structure, or cell sheet. The medium is preferably suitable for the maintenance of the cells in the sheet-like structure, and may be, for example, an MEM medium. After this operation, the medium may be further cultured at a temperature suitable for the proliferation of the Flk-1⁺ cells (step (6)). This operation is effective for the maintenance and proliferation of the Flk-1⁺ cells in the sheet-like structure (cell sheet), and prevents quality deterioration. The incubation temperature may be, for example, from 35°C to 38°C, and preferably 37°C. The sheet-like structure (cell sheet) collected from the culture vessel is usually transferred to another vessel as necessary, and stored immediately before use. The storage temperature is preferably low (for example, from 4°C to 15°C). Alternatively, the cell sheet may be subjected to transplantation without such storage (or prepared at the time of use).

### 2. IPS cell-derived vascular progenitor cell sheet

As described above, the inventors have succeeded in the construction of a vascular progenitor cell (Flk-1⁺ cell) sheet derived from iPS cells. The sheet thus obtained has a unique structure, and has a high utility value. Therefore, a second aspect of the present invention provides an iPS cell-derived vascular progenitor cell sheet defined by a unique structure (hereinafter abbreviated as "the cell sheet of the present invention"). In the cell sheet of the present invention, iPS cell-derived Flk-1⁺ cells form multiple layers, wherein the cells are embedded in a gel containing type I collagen, laminin, type IV collagen, and entactin. As a unique structure, the gel is present between the cells forming the cell layer. More specifically, basically, cells are not bonded or touching, but are intervened by a gel. This characteristic structure is found in at least 50% or more, preferably 70% or more, more preferably 90% or more, and most preferably 95% or more of the cell layers.

According to one embodiment, the cells composing the cell layer are Nanog⁻ cells. More specifically, the cell layers are composed of the iPS cell-derived Flk-1⁺ Nanog⁻ cells. In this manner, the use of the cells negative for the undifferentiated marker Nanog is important for the prevention of tumorigenesis after transplantation.

One of the characteristics of the cell sheet of the present invention is in that it includes multiple cell layers. Typically it includes 10 or more cell layers, specifically, for example, from 10 to 20 cell layers.

In a typical manner, the cell component in the cell layers is composed solely of iPS cell-derived Flk-1⁺ cells. More specifically, only the iPS cell-derived Flk-1⁺ cells compose the cell layers. According to one embodiment, the iPS cell-derived Flk-1⁺ cells and the cells derived from that Flk-1⁺ cells, or the cells developed by proliferation or differentiation of the iPS cell-derived Flk-1⁺ cells (for example, vascular endothelium precursor cells, vascular endothelial cells, vascular smooth muscle precursor cells, and vascular smooth muscle cells) compose the cell layers. The cell sheet is obtained by, for example, making a cell sheet including cell layers composed of iPS cell-derived Flk-1⁺ cells, and then culturing the sheet.

The cell sheet of the present invention can be obtained by, for example, the above-described production method of the present invention. In the cell sheet obtained by the production method of the present invention, the cells forming the cell layers are magnetically labeled. However, when the production method including the culture operation is used and proliferation of the cell occurs, the cells not magnetically labeled can be present.

### 3. Application of iPS cell-derived vascular progenitor cell sheet

The present invention further provides an angiogenesis therapy as a use of the iPS cell-derived vascular progenitor cell sheet. In the angiogenesis therapy of the present invention, the cell sheet obtained by the production method of the first aspect, or the cell sheet of the second aspect is transplanted to the affected part or injury part. Transplantation of the cell sheet promotes angiogenesis in the affected part or injury part. The present invention may be used for treatment of various diseases for which angiogenesis achieves therapeutic effect, for example, ischemic heart diseases (for example, angina pectoris and myocardial infarction), cerebrovascular disorders (for example, cerebral infarction and brain ischemia), obstructive arteriosclerosis, and critical inferior limb ischemia. In addition, the present invention may be used for promoting healing of wound and postoperative restoration of the injury part. For transplantation, as necessary, adhesion between the cell sheet and affected or injury part and/or integration of the cell sheet may be improved by seaming or using a biocompatible adhesive (for example, fibrin paste). However, the cell sheet used in the present invention is composed of cells embedded in a gel material including living body components, and has high adhesion properties and is expected to achieve a high integration rate. Accordingly, seaming or the use of an adhesive is not essential.

The treatment subject is not particularly limited, and include human and mammals other than human (including pet animals, livestock, and experimental animals; specific examples include mouse, rat, guinea pig, hamster, monkey, bovine, pig, goat, sheep, dog, cat, fowl, and quail). The treatment subject is preferably human.

### EXAMPLE

Vascular progenitor cells (VPCs) were induced to differentiate from mouse iPS cells, and these cells were further induced to differentiate into endothelial progenitor cells (EPCs) and vascular smooth muscle progenitor cells (SMPCs). Furthermore, in order to establish a novel revascularization/angiogenesis therapy, production of an iPS cell-derived vascular progenitor cell sheet was attempted.

### 1. Study of the method for inductive differentiation of vascular progenitor cells derived from iPS cells (ips vpc)

The mouse fetus fibroblast-derived iPS cells (ips-mef-ng-20D-17) (Takahashi K, Yamanaka S, Cell 2006, 126: 663-676.; Okita K, Ichisaka T, Yamanaka S, Nature 2007, 448: 313-317.) were cultured on a differentiation-inducing medium; Flk-1⁺ cells were found, and reproducible expression of Flk-1 was confirmed. In addition, differentiation of the iPS cell-derived Flk-1⁺ cells into endothelial cells and smooth muscle cells was confirmed. These cells were separately cultured, whereby a lumen forming network like a vascular endothelial cell was constructed. The inductive differentiation from iPS cells to Flk-1⁺ cells was carried out under the conditions described in the previous report (Circulation 2008, 118: 498-506.).

### 2. Study of safety and angiogenesis capacity of vascular progenitor cells derived from iPS cells (iPS VPS)

A model with limb ischemia was made using a nude mouse. iPS cell-derived Flk-1⁺ cells were transplanted to the ischemia side, and the ischemia improvement effect after limb ischemia was evaluated. As a result of this, the iPS cell-derived Flk-1⁺ cell transplant group showed a significant improvement in the blood flow on the limb ischemia side in comparison with the control group. In addition, the formation of organoid tumor was not found in any cell-transplanted groups up to 60 days after transplantation.

### 3. Making of iPS cell-derived vascular progenitor cell sheet

As shown in the above-described 1 and 2, angiogenesis effect was found in the Flk-1⁺ cells obtained from iPS cells. Therefore, as the next step, we started the development of a more efficient and effective cell transplantation method. In the course of the study, we focused on the magnetic engineering technology and collagen embedding method, and combined them to make the following method (see Fig. 1).

### (1) Magnetic label

iPS cell-derived Flk-1⁺ cells are suspended and floated in a microtube, and magnetic nanofine particles (MCL) are added thereto. The mixture is incubated at 37°C for 2 hours, and the MCLs are taken in the cells.

### (2) Preparation of gel material

Type I collagen (3 mg/ml), 10x MEM, a buffer solution (NaHCO₃) and FBS were mixed at a ratio of 7 : 1 : 1 : 1 (weight ratio) to make a collagen gel (1 ml contains 2.1 mg of type I collagen). Aside from this, a basement membrane gel containing laminin (560 mg/ml), type IV collagen (310 mg/ml) and entactin (80 mg/ml) is provided. In the following experiment, as the basement membrane gel , BD Matrigel (Japan Becton, Dickinson and Company) (composed of 56% of laminin, 31 % of type IV collagen, and 8% of entactin, and containing 0 to 0.1 pg/ml of bFGF, 0.5 to 1.3 ng/ml of EGF, 15.6 ng/ml of IGF-1, 12 pg/ml of PDGF, less than 0.2 ng/ml of NGF, and 2.3 ng/ml of TGF-β) was used.

### (3) Mixing of cells and gel material and plating

The magnetically labeled cells (the number of cells 1.7 x 10⁶ (100 μl), a collagen gel (170 μl), and a basement membrane gel (30 μl) were mixed, and plated on a ultra-low attachment culture dish (Corning).

### (4) Formation of cell layers by magnetic force

A magnet is placed on the bottom of the dish, magnetic force is applied, and the cells are drawn to the culture surface. When a cell layer is formed, redundant portions of the supernatant liquid are removed.

### (5) Gelation

The gel is incubated at 37°C for 1 hour to harden the gel. Thereafter, the medium is added.

As the iPS cell-derived Flk-1⁺ cells in (1), the Flk-1⁺ Nanog⁻ cells collected by FCM were used. The result of the analysis of the properties of the cells (expression profile of cell surface marker) is shown in Fig. 2.

As a result of the validation of the effectiveness of the above-described method, a sheet having a sufficient strength composed solely of iPS cell-derived Flk-1⁺ cells was successfully constructed. The cell sheet thus obtained was immunostained by an anti-Flk-1 antibody; Flk-1⁺ cells composed of 10 to 15 cell layers were observed (Fig. 3).

### 4. Study of safety and angiogenesis capacity of iPS cell-derived vascular progenitor cell sheet

A limb ischemia was made using a nude mouse. The iPS cell-derived FLk-1⁺ cell sheet was transplanted to the ischemia side (see Fig. 4), and the ischemia improvement effect after limb ischemia was evaluated by the laser Doppler method. As a comparative control, a cell sheet produced using the Flk-1⁻ cells derived from iPS cells (the production method is pursuant to the above-described (1) to (5)) was transplanted.

As shown in Fig. 5, The iPS cell-derived FLk-1⁺ cell sheet transplant group showed a significant improvement in the blood flow on the limb ischemia side on day 3, 7, 14, and 21 after operation in comparison with the iPS cell-derived Flk-1⁻ cell sheet transplant group and the control group. After transplantation of the iPS cell-derived Flk-1⁻ cell sheet, formation of organoid tumor was found at a high rate. In contrast, in the iPS cell-derived FLk-1⁺ cell sheet transplant group, formation of organoid tumor was not found up to 90 days after transplantation. In addition, formation of abundant blood vessels was found in the iPS cell-derived FLk-1⁺ cell sheet after transplantation (Fig. 6). The combination of the magnetic engineering technology and collagen embedding method allowed the formation of adequate gaps between the cells, whereby the formation of blood vessels in the multiple cell layers was enabled. The reason for this is likely that the blood supply into the cell sheet after transplantation prevented the death of the transplanted cells, which likely contributed to the improvement in the transplantation efficiency and integration ratio.

### 5. Validation of therapeutic effect of iPS cell-derived vascular progenitor cell sheet

A model with limb ischemia was made using a nude mouse. The iPS cell-derived FLk-1⁺ cell sheet was transplanted to the ischemia side, and the ischemia improvement effect after limb ischemia was evaluated by the laser Doppler method. As a comparative control, the cells used for the sheet formation (iPS cell-derived Flk-1⁺ cells) were transplanted (intramuscular injection).

As shown in Fig. 7A, the iPS cell-derived FLk-1⁺ cell sheet transplant group showed a significant improvement in the blood flow on the limb ischemia side in comparison with the cell transplant group on days 3, 7, 14, and 21 after operation. The tissues of the transplanted part were collected, and the expression level of various cytokines was measured; it was revealed that the expression of VEGF and bFGF important for angiogenesis was significantly higher in the iPS cell-derived FLk-1⁺ cell sheet transplant group (Fig. 7B). In addition, the TUNEL assay showed that cell death was significantly inhibited in the iPS cell-derived FLk-1⁺ cell sheet transplant group (Fig. 7c).

### 6. Study of rejection

The absence or presence of rejection after transplantation was evaluated using wild type mice of C57/B16 strain. An iPS cell-derived FLk-1⁺ cell sheet was transplanted to the adducent muscles of lower limbs of the mice, and histological comparison was carried out with the SHAM group. In addition, the expression level of inflammatory cytokine was also compared.

Some tissues were collected on day 21 after transplantation, and subjected to hematoxylin eosin staining; no rejection was found in the iPS cell-derived FLk-1⁺ cell sheet transplant group (Fig. 8A). In addition, no significant difference was found in the expression level of inflammatory cytokine IL-6 and MCP-1 between the Flk-1⁺ cell sheet transplant group and SHAM group (Figs. 8B and C). These results showed that the transplantation of the Flk-1⁺ cell sheet will not initiate rejection.

### 7. Apoptosis-inhibiting effect by magnetic label

The magnetically labeled iPS cell-derived Flk-1⁺ cells and iPS cell-derived Flk-1⁺ cells before magnetic labeling were provided, treated with BSO, and then subjected to trypan blue staining. The proportion of the trypan blue positive cells were lower in the magnetically labeled cells, indicating that the magnetic label (magnetic particles themselves) has apoptosis-inhibiting effect.

### INDUSTRIAL APPLICABILITY

According to the production method of the present invention, a cell sheet composed solely of iPS cell-derived vascular progenitor cells can be formed. At present, clinical application of iPS cells is attempted by many researchers, but one advantage of the use of iPS cells is that the iPS cell can be autotransplanted which cannot be achieved by ES cells and others. The cell sheet composed solely of iPS cell-derived vascular progenitor cells can use the peculiar advantage of iPS cells. In addition, this sheet is markedly superior to a cell sheet including adipocyte-derived stem cells in that it does not require the collection of adipose, and can be produced by a simple production process.

The cell sheet obtained by the production method of the present invention has a sufficient strength, includes multiple cell layers wherein adequate gaps are present between the cells, and allows transplantation with a high transplantation efficiency and a high integration ratio. The cell sheet is expected to find applications in the treatment of various diseases and clinical states to which angiogenesis achieves therapeutic effect.

The present invention will not be limited to the description of the embodiments and examples of the present invention. Various modifications readily made by those skilled in the art are also included in the present invention, without departing from the scope of claims. The entire contents of the articles, unexamined patent publications, and patent applications specified herein are hereby incorporated herein by reference.

SEQ ID NO.1 to 5: explanation of artificial sequence: adhesive peptide

## Claims

1. A method for producing an iPS cell-derived vascular progenitor cell sheet, comprising the following steps (1) to (4):
(1) a step for preparing magnetically labeled iPS cell-derived Flk-1⁺ cells;
(2) a step for plating a mixture of a gel material comprising type I collagen, laminin, type IV collagen, and entactin as active ingredients, and the Flk-1⁺ cells in a culture vessel;
(3) a step for drawing the Flk-1⁺ cells in the mixture to the culture surface in the culture vessel by application of a magnetic force to form multiple cell layers; and
(4) a step for gelling the gel material.

2. The production method of claim 1, wherein the step (1) comprises the following steps (1-1) to (1-4):
(1-1) a step for preparing iPS cells;
(1-2) a step for inducing differentiation of the iPS cells into Flk-1⁺ cells;
(1-3) a step for collecting Flk-1⁺ cells; and
(1-4) a step for magnetically labeling the collected Flk-1⁺ cells.

3. The production method of claim 2, wherein in the step (1-3), Nanog⁺ cells and Nanog⁻ cells are separated, and the Nanog⁻ Flk-1⁺ cells are collected.

4. The production method of any one of claims 1 to 3, wherein the mixture in the step (2) is obtained by mixing a first gel element composed of type I collagen as an active ingredient, a second gel element composed of laminin, type IV collagen, and entactin as active ingredients, and the Flk-1⁺ cells.

5. The production method of any one of claims 1 to 4, wherein an upwardly open section made by removable partitions is formed on the culture surface in the culture vessel in the step (2), and the mixture is plated in the section.

6. The production method of any one of claims 1 to 5, wherein the culture surface is low-adhesive.

7. The production method of any one of claims 1 to 6, wherein the step (3') is carried out between the steps (3) and (4):
(3') a step for removing the redundant part of the gel material from the upper part of the cell layer.

8. The production method of any one of claims 1 to 7, wherein the following step (5) is carried out after the step (4):
(5) a step for adding a medium to the culture vessel, and maintaining the sheet-like structure formed by the step in the medium; preferably wherein the following step (6) is carried out after the step (5):
(6) a step for culturing the Flk-1⁺ cells under temperature conditions which allow their growth.

9. A cell sheet obtained by the production method of any one of claims 1 to 8, wherein the cell sheet is composed of multiple layers of magnetically labeled iPS cell-derived Flk-1⁺ cells embedded in a gel containing type I collagen, laminin, type IV collagen, and entactin.

10. The cell sheet of claim 9, wherein the gel is present between the cells forming the multiple layers, preferably wherein the multiple layers comprise at least 10 layers, or wherein the multiple layers comprise 10 to 20 layers.

11. The cell sheet of claim 9 or 10, wherein the cell component contained in the multiple layers is composed solely of iPS cell-derived Flk-1⁺ cells, or wherein the cell component contained in the multiple layers is composed solely of the iPS cell-derived Flk-1⁺ cells and the cells derived from the cells.

12. The cell sheet of any one of claims 9 to 11 wherein the iPS cell-derived Flk-1⁺ cells are Nanog⁻ cells.

13. The cell sheet of any one of claims 9 to 12 for use in an angiogenesis therapy for transplantation to the affected or injury part, preferably for use in the healing of ischemic heart disease, cerebrovascular disorder, obstructive arteriosclerosis, critical inferior limb ischemia, or wound, or postoperative healing of wound.

## Patentansprüche

1. Verfahren zur Herstellung einer von einer iPS-Zelle stammenden vaskulären Vorläuferzellschicht umfassend die folgenden Schritte (1) bis (4):
(1) Schritt zur Herstellung magnetisch markierter von einer iPS-Zelle stammenden Flk-1⁺-Zellen;
(2) Schritt zur Plattierung einer Mischung eines Gelmaterials umfassend Typ I Kollagen, Laminin, Typ IV Kollagen und Entactin als Wirkstoffe, sowie die Flk-1⁺-Zellen in einem Kulturgefäß;
(3) Schritt zum Ziehen der Flk-1⁺-Zellen in der Mischung an die Kulturoberfläche in dem Kulturgefäß durch Anwendung einer magnetischen Kraft, um zahlreiche Zellschichten zu bilden;
und
(4) Schritt zur Gelierung des Gelmaterials.

2. Herstellungsverfahren nach Anspruch 1, wobei der Schritt (1) die folgenden Schritte (1-1) bis (1-4) umfasst:
(1-1) Schritt zur Herstellung von iPS-Zellen;
(1-2) Schritt zur Induktion der Differenzierung der iPS-Zellen in Flk-1⁺-Zellen;
(1-3) Schritt zur Sammlung von Flk-1⁺-Zellen; und
(1-4) Schritt zur magnetischen Markierung der gesammelten Flk-1⁺-Zellen.

3. Herstellungsverfahren nach Anspruch 2, wobei in dem Schritt (1-3) Nanog⁺-Zellen und Nanog⁻-Zellen separiert werden, und die Nanog⁻-Flk-1⁺-Zellen gesammelt werden.

4. Herstellungsverfahren nach einem der Ansprüche 1 bis 3, wobei die Mischung aus Schritt (2) durch Mischen eines ersten Gelelementes, das sich aus Typ I Kollagen als Wirkstoff zusammensetzt, eines zweiten Gelelements, das sich aus Laminin, Typ IV Kollagen und Entactin als Wirkstoffe zusammensetzt, und der Flk-1⁺-Zellen erhalten wird.

5. Herstellungsverfahren nach einem der Ansprüche 1 bis 4, wobei ein nach oben geöffneter Bereich, der durch entfernbare Abtrennungen hergestellt wird, an der Kulturoberfläche in dem Kulturgefäß in dem Schritt (2) gebildet wird, und die Mischung in den Bereich plattiert wird.

6. Herstellungsverfahren nach einem der Ansprüche 1 bis 5, wobei die Kulturoberfläche gering adhäsiv ist.

7. Herstellungsverfahren nach einem der Ansprüche 1 bis 6, wobei der Schritt (3') zwischen den Schritten (3) und (4) durchgeführt wird:
(3') Schritt zur Entfernung des überflüssigen Teils des Gelmaterials von dem oberen Teil der Zellschicht.

8. Herstellungsverfahren nach einem der Ansprüche 1 bis 7, wobei der folgende Schritt (5) nach dem Schritt (4) durchgeführt wird:
(5) Schritt zur Hinzufügung eines Mediums zu dem Kulturgefäß und Beibehalten der schichtähnlichen Struktur in dem Medium, die durch den Schritt gebildet wird; vorzugsweise wobei der folgende Schritt (6) nach dem Schritt (5) durchgeführt wird:
(6) Schritt zur Kultivierung der Flk-1⁺-Zellen unter Temperaturbedingungen, die deren Wachstum erlauben.

9. Zellschicht, die durch das Herstellungsverfahren nach einem der Ansprüche 1 bis 8 erhalten wird, wobei die Zellschicht aus zahlreichen Schichten magnetisch markierter von einer iPS-Zelle stammenden Flk-1⁺-Zellen zusammengesetzt ist, die in einem Gel enthaltend Typ I Kollagen, Laminin, Typ IV Kollagen und Entactin eingebettet sind.

10. Zellschicht nach Anspruch 9, wobei sich das Gel zwischen den Zellen, die die zahlreichen Schichten bilden, befindet, vorzugsweise wobei die zahlreichen Schichten mindestens 10 Schichten umfassen, oder wobei die zahlreichen Schichten 10 bis 20 Schichten umfassen.

11. Zellschicht nach Anspruch 9 oder 10, wobei der in den zahlreichen Schichten enthaltene Zellbestandteil ausschließlich aus von einer iPS-Zelle stammenden Flk-1⁺-Zellen zusammengesetzt ist, oder wobei der in den zahlreichen Schichten enthaltene Zellbestandteil ausschließlich aus von einer iPS-Zelle stammenden Flk-1⁺-Zellen und den Zellen, die von den Zellen abstammen, zusammengesetzt ist.

12. Zellschicht nach einem der Ansprüche 9 bis 11, wobei die von einer iPS-Zelle stammenden Flk-1⁺-Zellen Nanog⁻-Zellen sind.

13. Zellschicht nach einem der Ansprüche 9 bis 12 zur Verwendung in einer Angionese-Therapie zur Transplantation in den betroffenen oder verletzten Teil, vorzugsweise zur Verwendung in der Heilung von einer ischämischen Herzkrankheit, einer zerebrovaskulären Erkrankung, einer obstruktiven Arteriosklerose, einer kritischen Ischämie der unteren Gliedmaßen, oder einer Wunde, oder die postoperative Heilung einer Wunde.

## Revendications

1. Procédé de production d'une feuille de cellules progénitrices vasculaires issues de cellules iPS, comprenant les étapes (1) à (4) suivantes:
(1) une étape de préparation de cellules Flk-1⁺ issues de cellules iPS marquées magnétiquement;
(2) une étape de mise en plaque d'un mélange d'un matériau de gel comprenant du collagène de type I, de la laminine, du collagène de type IV et de l'entactine en tant qu'ingrédients actifs, et les cellules Flk-1⁺ dans un récipient de culture;
(3) une étape d'attraction des cellules Flk-1⁺ dans le mélange vers la surface de la culture dans le récipient de culture par application d'une force magnétique pour former multiples couches de cellules; et
(4) une étape de gélification du matériau de gel.

2. Procédé de production selon la revendication 1, dans lequel l'étape (1) comprend les étapes (1-1) à (1-4) suivantes:
(1-1) une étape de préparation de cellules iPS;
(1-2) une étape d'induction d'une différenciation des cellules iPS en cellules Flk-1+;
(1-3) une étape de récupération des cellules Flk-1+; et
(1-4) une étape de marquage magnétique des cellules Flk-1⁺ recueillies.

3. Procédé de production selon la revendication 2, dans lequel, dans l'étape (1-3), des cellules Nanog⁺ et des cellules Nanog⁻ sont séparées, et les cellules Flk-1⁺ Nanog⁻ sont recueillies.

4. Procédé de production selon l'une quelconque des revendications 1 à 3, dans lequel le mélange dans l'étape (2) est obtenu par mélange d'un premier élément de gel composé de collagène de type I en tant qu'ingrédient actif, d'un second élément de gel composé de laminine, de collagène de type IV et d'entactine en tant qu'ingrédients actifs, et des cellules Flk-1⁺.

5. Procédé de production selon l'une quelconque des revendications 1 à 4, dans lequel une section ouverte vers le haut réalisée par des partitions amovibles est formée sur la surface de culture dans le récipient de culture dans l'étape (2), et le mélange est mis en plaque dans la section.

6. Procédé de production selon l'une quelconque des revendications 1 à 5, dans lequel la surface de culture est faiblement adhésive.

7. Procédé de production selon l'une quelconque des revendications 1 à 6, dans lequel l'étape (3') est effectuée entre les étapes (3) et (4):
(3') une étape d'élimination de la partie redondante du matériau de gel de la partie supérieure de la couche de cellules.

8. Procédé de production selon l'une quelconque des revendications 1 à 7, dans lequel l'étape (5) suivante est effectuée après l'étape (4):
(5) une étape d'addition d'un milieu dans le récipient de culture et le maintien de la structure en feuille formée par l'étape dans le milieu; de préférence dans lequel l'étape (6) suivante est effectuée après l'étape (5);
(6) une étape de culture des cellules Flk-1⁺ dans des conditions de température qui permettent leur croissance.

9. Feuille de cellules obtenue par le procédé de production selon l'une quelconque des revendications 1 à 8, dans laquelle la feuille de cellules est composée de multiples couches de cellules Flk-1⁺ issues de cellules iPS marquées magnétiquement noyées dans un gel contenant du collagène de type I, de la laminine, du collagène de type IV et de l'entactine.

10. Feuille de cellules selon la revendication 9, dans laquelle le gel est présent entre les cellules formant les multiples couches, de préférence dans laquelle les multiples couches comprennent au moins 10 couches, ou dans laquelle les multiples couches comprennent de 10 à 20 couches.

11. Feuille de cellules selon la revendication 9 ou 10, dans laquelle le composant cellule contenu dans les multiples couches est uniquement composé des cellules Flk-1⁺ issues de cellules iPS, ou dans laquelle le composant cellule contenu dans les multiples couches est uniquement composé des cellules Flk-1⁺ issues de cellules iPS et des cellules issues des cellules.

12. Feuille de cellules selon l'une quelconque des revendications 9 à 11, dans laquelle les cellules Flk-1⁺ issues de cellules iPS sont des cellules Nanog⁻.

13. Feuille de cellules selon l'une quelconque des revendications 9 à 12, pour une utilisation dans une thérapie d'angiogénèse pour la transplantation dans la partie affligée ou blessée, de préférence pour une utilisation dans la cicatrisation d'une maladie cardiaque ischémique, d'un trouble cérébrovasculaire, de l'artériosclérose obstructrice, d'une ischémie critique des membres inférieurs ou d'une plaie, ou dans la cicatrisation post-opératoire d'une plaie.


### SEQUENCE LISTING

<110> NATIONAL UNIVERSITY CORPORATION NAGOYA UNIVERSITY
   MUROHARA, Toyoaki
   HONDA, Hiroyuki

   SHIBATA, Rei
   ISHII, Masakazu
   KITO, Tetsutaro
   SUZUKI, Hirohiko
<120> iPS cell-derived vascular progenitor cell sheet and method for producing the same
<130> NU11007P
<150> JP P2011-244046
   <151> 2011-11-08
<160> 5
<170> PatentIn version 3.5
<210> 1
   <211> 6
   <212> PRT
   <213> Artificial Sequence
<220>
   <223> adhesive peptide
<400> 1
   Lys Gln Ala Gly Asp Val
   1               5
<210> 2
   <211> 4
   <212> PRT
   <213> Artificial Sequence
<220>
   <223> adhesive peptide
<400> 2
   Arg Glu Asp Val
   1
<210> 3
   <211> 5
   <212> PRT
   <213> Artificial Sequence
<220>
   <223> adhesive peptide
<400> 3
   Tyr Ile Gly Ser Arg
   1               5
<210> 4
   <211> 5
   <212> PRT
   <213> Artificial Sequence
<220>
   <223> adhesive peptide
<400> 4
   Ile Lys Val Ala Val
   1               5
<210> 5
   <211> 4
   <212> PRT
   <213> Artificial Sequence
<220>
   <223> adhesive peptide
<400> 5
   Arg Gly Asp Cys
   1